# EUROPEAN PATENT SPECIFICATION

(11) **EP 0 847 250 B1**
(45) Date of publication and mention of the grant of the patent: **30.07.2003**
(21) Application number: 97928439.5
(22) Date of filing: 25.06.1997
(51) Int. Cl.: A61B 17/04

(54) **SUTURE BLOCK FOR SURGICAL SUTURES**
KLEMMVORRICHTUNG FÜR CHIRURGISCHE NÄHFÄDEN
BLOC DE SUTURE POUR SUTURES CHIRURGICALES

(30) Priority: 27.06.1996 IT RM960451
(43) Date of publication of application: 17.06.1998
(73) Proprietor: Valenti, Gabriele, 00153 Roma (IT)
(72) Inventor: Valenti, Gabriele, 00153 Roma (IT)
(74) Representative: Lanzoni, Luciano
(86) International application number: IT9700150
(87) International publication number: WO97049341

(56) References cited:
- EP-A- 0 012 360
- EP-A- 0 594 002
- EP-A- 0 634 142
- WO-A-94/15535
- FR-A- 2 682 867
- US-A- 2 075 508
- US-A- 3 753 438
- US-A- 3 976 079
- US-A- 5 258 015

## Description

A proposal for a suture block for surgical sutures.

This carefully-devised invention is made of re-absorbable, non-re-absorbable, metallic or a blend of materials, just like normal two-part surgical sutures, which are joined together and secured to prevent the thread from coming undone.

Apart from the traditional knot, the options which exist today as regards securing sutures, consist of metal clips or clips made of materials which are gradually reabsorbed. Clips, however, block the thread in the acute angle formed by the two linear parts before they are closed - in other words, in the vertex of the "V". Yet this an unsuitable and imbalanced position since the thread is subjected to traction and tension. Thus, instead of being and remaining in a perfectly orthogonal position in the direction of the traction, the clip tends to assume a vertical position, putting the tissue at one end under pressure and losing optimum surface contact - and hence much of the stability needed for it to serve its purpose. What's more, the linear form of the clip, once closed, is completely ineffectivetl in achieving the desired effect. Invented to hold vessels or tissue together, it is used incorrectly to secure threads. I should add that the use of metal clips entails the use of excessive amounts of material - something which the patent finds hard to accept and which, moreover, interferes with certain diagnostic techniques. Clips made of re-absorbable material do not grip the thread and pose the same problems in terms of stability and ergonomics.

Even the recent introduction of the Lapra-Ty suture block, which addresses the problem, does not entirely solve it. In practice, this invention works on the basis of the same principle as a normal clip, already outlined, combined with an eccentric clasp and stop, which thus give rise to its shortcomings, namely, its instability and poor ergonomics. The only difference is that it is thicker than a normal clip and thus relies on this increased contact surface area, in order to secure the thread. The manufacturers themselves suggest that it only be used with woven threads, i.e. with those which may be compressed and flattened, and which have a limited diameter - in other words, least resistant ones.

Another method of securing a thread is by passing it through a little bead followed by a small lead ring, which works like a cork and a lead pellet. This method which is used only for cutaneous sutures, has several drawbacks. Firstly, the surgeon is forced to thread the needle first through the bead and then through the lead pellet, which must finally be secured using a surgical instrument: secondly, there is the risk that the needle holder lose its grip, and having to change instrument is a further waste of time. Besides, the threads available on the market are meant for continuous sutures and a similar procedure for sutures consisting of isolated stitches would be unthinkable, due to the sheer number of beads and lead pellets required, and the risk of confusion on the operating table, which could easily result Lastly, the sphere which functions as a suture block and which is positioned between the tissue and the lead pellet, in theory only has punctiform contact with the tissue, when full surface contact is what is really required. In fact, contact between the sphere and the tissue plane only occurs at the tangential point.

The result here too is therefore also unsatisfactory: in practice, the larger the contact surface area, the less is the pressure when equal force is applied, and, subsequently, the minor the damage done by the suture block to the tissue. Another method is shown in the patent US A 3,976,079, which discloses a suture block for holding surgical sutures comprising a body of essentially discoidal shape including a through cavity, a peg insertable into said cavity in an insertion direction, said discoidal body including an opening for passage and positioning, transverse to the insertion direction of the peg, of the suture thread into said cavity, thereby to co-operate with the peg to block the suture thread through a forced insertion of the peg and the thread into said cavity in the insertion direction.

The fact that none of these methods are used as a matter of course as a way to avoid tying knots, proves the point. In laparoscopic surgery the same problem exists - all the methods used to secure a suture are awkward and imprecise.

Too many problems and risks are therefore involved, using clips for a different purpose from which they were intended, makes the surgeon feel uneasy, and the use of beads and lead pellets, is too complicated, empiric and haphazard.

The innovations introduced with this invention are aimed at offering the surgeon with a new, quicker suture technique, which is less painful and less traumatising for the tissue, and, on occasions, easier to perform.
* The first characteristic of the invention is its disc-shaped form which provides the optimal surface area for contact with the tissue, whilst using the minimum of material. It thereby distributes the tension of the thread uniformly over the largest possible surface area.
* The second characteristic of the invention is that it enables the thread to be passed through the centre of the disc thus saving time and , should there be traction, provides balance and stability, guaranteeing optimal, uniform contact between the surface of the disc and the tissue.
* Another characteristic of the invention is its suture blocking system which prevents the thread from working itself loose and at the same time does not weaken it , which is fundamental given the tension to which it is regularly subjected.
* Another caharacteristic of the invention is that it has a peg - the "male" part - which thanks to a labyrinthine system, gradually blocks the thread whatever its thickness (within, of course, the limits of the calibres and materials normally used in surgery).
* Another characteristic of the invention is that it exploites the elasticity and plasticity of the materials normally used in surgery in order to obtain a suture block fashioned in such a way that as the peg gradually enters its female counterpart, the walls "give" because of their elastomeric properties, both in order to hold the thread in place and to adapt to its variable diameter, without compressing it to such an extent that its resistance is reduced.
* Another characteristics of the invention is that the front face of the male peg has what could be described as a "V"- shaped mouth, which serves both to position the thread within the suture block, and to ensure that as the thread is introduced, it is gradually tightened, irrespective of its thickness, by a self-blocking system which is independent from the above-mentioned labyrinthine mechanism. The self-blocking system is the result of the combined effect of the "V"-shaped mouth, and the light pressure to which the two jaws of the "V" are subjected as the suture stop is closed. It works in the same way as the sheet pins on a yacht.
* Another characteristic of the invention is that the male part is locked into the shell by a press-stud, spring toggle, or a mixed system.
* Another characteristic of the invention is that its shell may consist of two separate pieces, or may be a single system in which the parts are hinged together, or attached to each other by a film.
* Another characteristtic of the invention is that it has been designed in such a way as to ensure that the thread is always clamped at the centre of the system..
* Another characteristic of the invention is that a suture which is bound to the tissue is obtained, without the latter being constricted by a knot. This lowers the risk of eschemia, reduces pain and limits tissue trauma and damage.
* Another characteristic of the invention is that the edges are rounded and so they reduce the damage that could be caused should they come into contact with the tissue.
* Another characteristic of the invention is the ease, simplicity and speed with which it may be applied, reducing the complexity, length and cost of the surgical operation.
* Another characteristic of the invention is that once the disc has been secured at the end of a continuous or isolated - stitch suture, by applying a second disc close to the previous one and then cutting the thread between the two, the suture can be made ready for the next stitch, with the suture block already in place at the end of the disc.
* Another characteristic of the invention is that its saves on the large quantities of sutural thread normally required.
* Another characeristic of the invention is that it can be applied independently, or with disposable loaders, as is the case with clips.
* Another characteristic of the invention is to offer the surgeon the possibility of performing a suture involving several stitches, without having to change the disposition of the operational field and without having to put down his instruments to free his hands to tie the knots.

With reference to the Figures, the number 1 indicates a suture block for surgical sutures made of re-absorbable or non re-absorbable material.

Specifically, the suture block 1 comprises a body 2 of essentially discoidal shape having a through cavity 4. The number 5 indicates a peg which is destined to be stably coupled by forced insertion into the cavity 4 through forced fastening means. Advantageously the peg 5 presents a longitudinal through slot 12 to increase its elastic characteristics.

The discoidal body 2 presents centrally an opening 6 for the passage and the transverse positioning into said cavity 4 of a suture thread.

The opening 6 is destined to co-operate with a V-shaped opening 7, elastically deformable, presented by the front end of the peg 5 centring and blocking the suture thread, whatever its thickness, through the forced insertion of the peg 5 into said cavity 4.

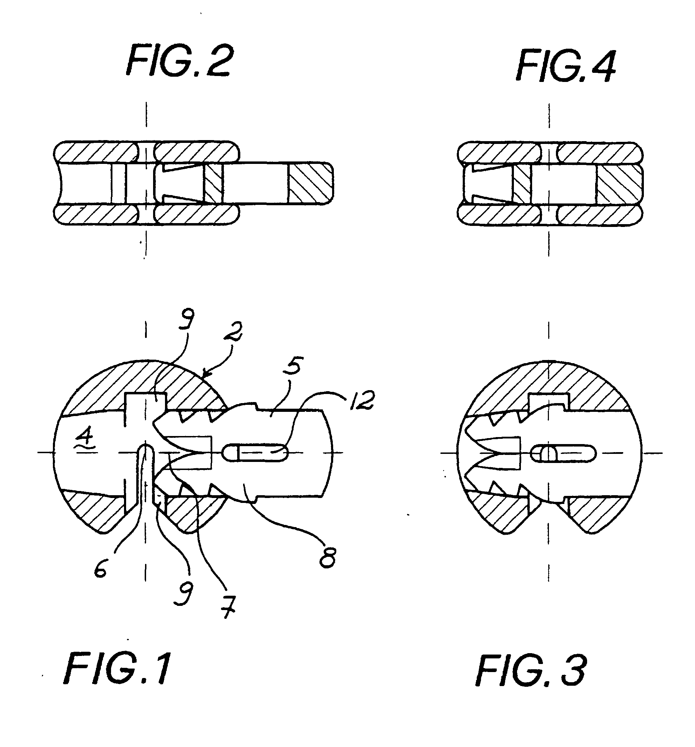

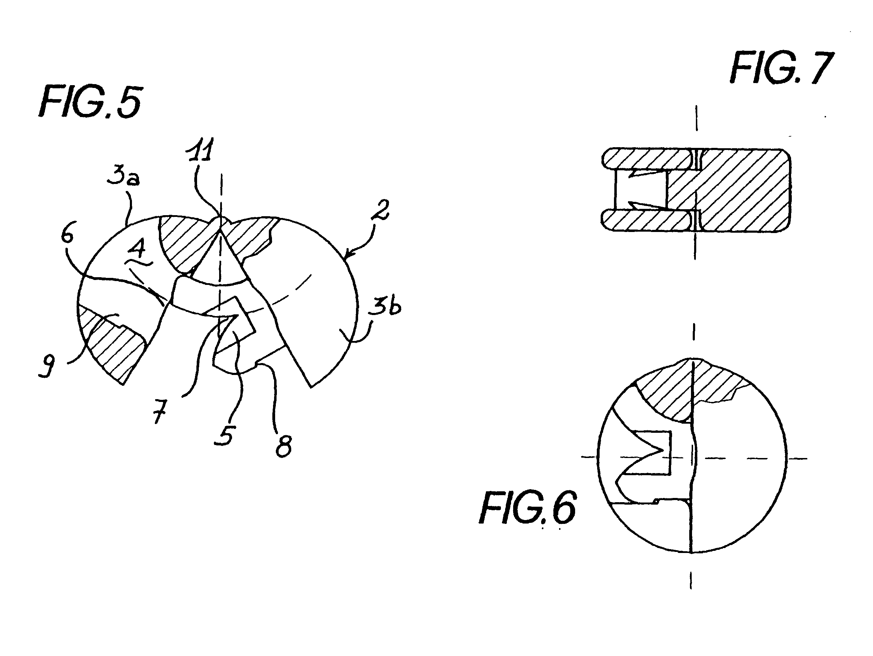

As shown in Figures 1 and 5, the forced blocking means comprise at least one tooth 8 presented laterally by the peg 5 snapping, at the end of the forced insertion, in at least one corresponding recess 9 presented by the inner surface of the cavity 4.

As shown in particular in Figures 1, 2, 3, 4, the cavity 4 passes through the entire discoidal body 2. The opening 6 for the passage and the positioning of the suture thread is obtained orthogonally to said cavity 4, from the periphery to the centre of the discoidal body 2.

Also in the Figures it is shown that the peg 5 is inserted from the outside of the discoidal body 2 by forced insertion into said cavity 4 and it presents on both its sides multiple teeth 8 which are destined to snap in opposing recesses 9 presented by the inner surface of the cavity 4.

As shown in Figures 5, 6, 7 the discoidal body 2 comprises two semi-discoidal parts mutually connected at an end by a hinge element 11, wherein one of said semi-discoidal parts 3b presents integrally in correspondence with its inner surface the peg 5 which is destined to be inserted by forced insertion into the cavity 4 is obtained centrally in the other semi-discoidal part 3a by partial convergent rotation of said semi-discoidal parts 3a, 3b.

The opening 6 for the passage and positioning of the suture thread is obtained from opposite conforming central concavities obtained on the opposing inner surfaces of said two semi-discoidal parts 3a, 3b.

Advantageously as shown in all figures the discoidal body 2 presents rounded peripheral edges.

Naturally, the present invention can be subject to numerous modifications and variations, without thereby departing from the scope of the appended claims.

## Claims

1. A suture block for holding a surgical suture comprising:
a body (2) of essentially discoidal shape, including a through cavity (4) in at least one part (3a, 3b) thereof;
a peg (5) insertable into said cavity (4) in an insertion direction and including a V-shaped opening at a front end thereof;
said discoidal body (2) including an opening (6) for passage and positioning, transverse to the insertion direction, of the suture thread into said cavity (4), thereby to co-operate with the V-shaped opening (7) elastically deformable to center and block the suture thread, whatever its thickness, through a forced insertion of the peg (5) and the thread into said cavity (4) in the insertion direction;
said peg (5) including at least one tooth (8) laterally fastening the peg by snapping, at the end of the forced insertion, in at least one corresponding recess (9) of an inner surface of the cavity (4).

2. Suture block for surgical sutures, according to claim 1, **characterised in that** said cavity (4) passes through the entire discoidal body (2) and **in that** said opening (6) for the passage and the positioning of the suture thread is disposed orthogonally to said cavity (4) and the insertion direction, from the periphery to the center of the discoidal body (2), said peg (5) being inserted from the outside in the discoidal body (2) by the forced insertion into said cavity (4) and including on both its sides multiple teeth (8) destined to snap in opposing recesses (9) presented by the inner surface of the cavity (4).

3. Suture block for surgical sutures, according to claim 2 **characterised in that** said peg (5) includes a through longitudinal slot (12) for increasing the elastic characteristics of the peg (5).

4. Suture block for surgical sutures, according to claim 1, **characterised in that** said discoidal body (2) comprises two semi-discoidal parts (3a, 3b) mutually connected at an end by a hinge element (11), wherein one of said semi-discoidal parts (3b) presents integrally in correspondence with its inner surface the peg (5) destined to be inserted by forced insertion into the cavity (4) obtained centrally in the other semi-discoidal part (3a) by partial convergent rotation of said semi-discoidal parts (3a, 3b).

5. Suture block for surgical sutures, according to claim 4, **characterised in that** said opening (6) for the passage and positioning of the suture thread is obtained from opposite conforming central concavities obtained on the opposing inner surfaces of said two semi-discoidal parts (3a, 3b).

6. Suture block for surgical sutures, according to claim 1, **characterised in that** the discoidal body (2) presents rounded peripheral edges.

7. Suture block for surgical sutures, according to claim 1, **characterised in that** it is manufactured in re-absorbable material.

8. Suture block for surgical sutures, according to claim 1, **characterised in that** it is manufactured in non re-absorbable material.

## Patentansprüche

1. Klemmvorrichtung für chirurgische Nähfäden, enthaltend:
- einen Körper (2) von im wesentlichen scheibenförmiger Ausführung, der wenigstens in einem seinem Teil (3a, 3b) einen durchgehenden Hohlraum (4) enthält;
- einen in einer Einschubrichtung in den genannten Hohlraum (4) einsetzbaren Stift (5), enthaltend eine V-förmige Öffnung an seinem vorderen Ende;
wobei der genannte scheibenförmige Körper (2) eine Öffnung (6) zum Durchführen und Positionieren des Nähfadens in dem genannten Hohlraum (4) quer zu der Einschubrichtung enthält, die mit der elastisch verformbaren, V-förmigen Öffnung (7) zusammenarbeitet, um den Nähfaden zu zentrieren und festzuklemmen, unabhängig von dessen Stärke, und zwar durch ein forciertes Einschieben des Stiftes (5) und des Nähfadens in den genannten Hohlraum (4) in Einschubrichtung;
wobei der genannte Stift (5) wenigstens einen Zahn (8) aufweist, welcher den Stift nach dem forcierten Einschieben durch Einschnappen in wenigstens eine entsprechende Aussparung (9) seitlich an der inneren Oberfläche des Hohlraumes (4) befestigt.

2. Klemmvorrichtung für chirurgische Nähfäden nach Patentanspruch 1, **dadurch gekennzeichnet, dass** der genannte Hohlraum (4) durch den gesamten scheibenförmigen Körper (2) verläuft, und dadurch, dass die genannte Öffnung (6) zum Durchführen und Positionieren des Nähfadens rechtwinklig zu dem genannten Hohlraum (4) und zu der Einschubrichtung angeordnet ist, und zwar vom Umfang aus zur Mitte des scheibenförmigen Körpers (2) hin, wobei der genannte Stift (5) durch das forcierte Einschieben in den genannten Hohlraum (4) von aussen in den scheibenförmigen Körper (2) eingesetzt wird, und wobei er an beiden seiner Seiten mehrere Zähne (8) enthält, dazu bestimmt, in die sich gegenüberliegenden Aussparungen (9) einzuschnappen, die von den inneren Oberflächen des Hohlraumes (4) aufgewiesen werden.

3. Klemmvorrichtung für chirurgische Nähfäden nach Patentanspruch 2, **dadurch gekennzeichnet, dass** der genannten Stift (5) einen durchgehenden, länglichen Schlitz (12) aufweist, um die elastischen Eigenschaften des Stiftes (5) zu erhöhen.

4. Klemmvorrichtung für chirurgische Nähfäden nach Patentanspruch 1, **dadurch gekennzeichnet, dass** der genannte scheibenförmige Körper (2) zwei halbscheibenförmige Teile (3a, 3b) enthält, die an einem Ende durch ein Scharnierelement (11) miteinander verbunden sind, wobei eins der genannten halbscheibenförmigen Teile (3b) fest mit seiner inneren Oberfläche verbunden den Stift (5) aufweist, der dazu bestimmt ist, durch forciertes Einschieben in den Hohlraum (4) eingesetzt zu werden, der sich in dem anderen halbscheibenförmigen Teil (3a) befindet, und zwar durch eine teilweise konvergierende Umdrehung der genannten halbscheibenförmigen Teile (3a, 3b).

5. Klemmvorrichtung für chirurgische Nähfäden nach Patentanspruch 4, **dadurch gekennzeichnet, dass** die genannten Öffnung (6) zum Durchführen und Positionieren des Nähfadens aus sich gegenüberliegenden und übereinstimmenden mittleren Vertiefungen gebildet ist, erhalten an den einander zugewandten inneren Oberflächen der genannten beiden halbscheibenförmigen Teile (3a, 3b).

6. Klemmvorrichtung für chirurgische Nähfäden nach Patentanspruch 1, **dadurch gekennzeichnet, dass** der scheibenförmige Körper (2) abgerundete umlaufende Kanten aufweist.

7. Klemmvorrichtung für chirurgische Nähfäden nach Patentanspruch 1, **dadurch gekennzeichnet, dass** sie aus einem resorptionsfähigen Material hergestellt ist.

8. Klemmvorrichtung für chirurgische Nähfäden nach Patentanspruch 1, **dadurch gekennzeichnet, dass** sie nicht aus einem resorptionsfähigen Material hergestellt ist.

## Revendications

1. Bloc de suture pour retenir une suture chirurgicale comprenant:
- un corps (2) essentiellement en forme de disque, comprenant une cavité de passage (4) dans au moins une de ses parties (3a, 3b);
- une cheville (5) insérable dans ladite cavité (4) dans une direction d'insertion et comprenant une ouverture en forme de "V" à son extrémité avant;
- ledit corps en forme de disque (2) comprenant une ouverture (6) pour le passage et la mise en place, transversale à la direction d'insertion, d'un fil de suture dans ladite cavité (4), de manière à coopérer avec l'ouverture en forme de "V" (7) élastiquement déformable pour centrer et bloquer le fil de suture, indépendamment de son épaisseur, à travers une insertion forcée de la cheville (5) et du fil dans ladite cavité (4) dans la direction d'insertion;
- ladite cheville (5) comprenant au moins une dent (8) fixant latéralement la cheville par frottement à force, à la fin de l'insertion forcée, dans au moins un évidement correspondant (9) d'une surface intérieure de la cavité (4).

2. Bloc de suture pour sutures chirurgicales selon la revendication 1, **caractérisé en ce que** ladite cavité (4) passe à travers tout le corps en forme de disque (2) et **en ce que** ladite ouverture (6) pour le passage et la mise en place du fil de suture est disposée orthogonale à ladite cavité (4) et à la direction d'insertion, de la périphérie vers le centre du corps en forme de disque (2), ladite cheville (5) étant insérée de l'extérieur dans le corps en forme de disque (2) par insertion forcée dans ladite cavité (4) et présentant sur ses deux côtés des dents multiples (8) destinées à s'insérer par frottement à force dans des évidements opposés (9) présentés par la surface intérieure de la cavité (4).

3. Bloc de suture pour sutures chirurgicales selon la revendication 2, **caractérisé en ce que** ladite cheville (5) comporte une fente longitudinale de passage (12) pour augmenter les caractéristiques élastiques de la cheville (5).

4. Bloc de suture pour sutures chirurgicales selon la revendication 1, **caractérisé en ce que** ledit corps en forme de disque (2) comporte deux parties en semi-disque (3a, 3b) reliées réciproquement à une extrémité par un élément à charnière (11), dans lequel une desdites parties en semi-disque (3b) présente de manière solidaire en correspondance avec sa surface intérieure la cheville (5) destinée à être insérée par insertion à force dans la cavité (4) obtenue au centre dans l'autre partie en semi-disque (3a) par rotation convergente partielle desdites parties en semi-disque (3a, 3b).

5. Bloc de suture pour sutures chirurgicales selon la revendication 4, **caractérisé en ce que** ladite ouverture (6) pour le passage et la mise en place du fil de suture est réalisée par des concavités centrales opposées correspondantes obtenues sur les surfaces intérieures opposées desdites deux parties en semi-disque (3a, 3b).

6. Bloc de suture pour sutures chirurgicales selon la revendication 1, **caractérisé en ce que** ledit corps en forme de disque (2) présente des bords périphériques arrondis.

7. Bloc de suture pour sutures chirurgicales selon la revendication 1, **caractérisé en ce qu'**il est fabriqué en matériau susceptible d'être réabsorbé.

8. Bloc de suture pour sutures chirurgicales selon la revendication 1, **caractérisé en ce qu'**il est fabriqué en un matériau qui n'est pas susceptible d'être réabsorbé.
